# EUROPEAN PATENT APPLICATION

(11) **EP 4 563 154 A1**
(43) Date of publication of application: **04.06.2025**
(21) Application number: 23846715.3
(22) Date of filing: 31.07.2023
(51) Int. Cl.: A61K 33/00, A61K 9/72, A61P 1/00, A61P 13/02, A61P 25/28

(54) **HYDROGEN-GAS-CONTAINING DRUG FOR CAUSAL TREATMENT OF ALZHEIMER'S DISEASE (DISEASE-MODIFYING DRUG)**

(30) Priority: 29.07.2022 JP 2022122081
(71) Applicant: H2 Global Group s.r.o., Muglinov 712 00 Ostrava (CZ)
(72) Inventor: ONO Hirohisa, Numazu-shi, Shizuoka 4100022 (JP); NISHIJIMA Yoji, Numazu-shi, Shizuoka 410-0022 (JP); OHTA Shigeo, Kawasaki-shi, Kanagawa 211-0004 (JP)
(74) Representative: Lichtnecker, Markus Christoph
(86) International application number: PCT/JP2023/028004
(87) International publication number: WO 2024/024985

(57) **Abstract**

Provided is a drug for treating Alzheimer's disease, the drug enabling retention of cognitive function amelioration and nerve quality improvement for a specific time even after treatment ends. This drug for causal treatment of Alzheimer's disease (disease-modifying drug) contains hydrogen gas as an active ingredient.

## Description

### Field of Invention

The present invention relates to a composition for use in the causal treatment of progressive forms of Alzheimer's disease.

### Background Art

Alzheimer's disease (AD) is a progressive neurodegenerative disease and the most common cause of dementia. It causes significant cognitive decline, leading to memory loss and impairment of daily activities. In the final stages of the disease, the affected patient loses the ability to respond to his or her environment, carry on a conversation, and control movement, leading to the patient's death.

With continued deterioration in memory and cognitive function, serious personality changes can occur and the individual needs extensive care. Urinary and faecal incontinence is an accompanying symptom of the inevitable reduction in quality of life in the progressive form of Alzheimer's disease. Treatments currently being developed for Alzheimer's disease use improvement in memory function as an indicator of improvement, but do not assess improvement in other Alzheimer's-related difficulties as determinants of quality of life.

Molecular hydrogen (molecular hydrogen formula H₂, hereafter referred to as "hydrogen") was first described as an antioxidant with medicinal uses. Subsequent research has shown that hydrogen has multiple functions that are effective in different disease models. Hydrogen gas has been shown to be beneficial in the elderly patients in stroke, heart attack, chronic obstructive pulmonary disease, cancer and COVID-19. Importantly, the safety of hydrogen gas inhalation has been confirmed in Phase I clinical trials. Subgroup analysis of randomized clinical trials indicates that hydrogen improved the condition of test subjects with the apolipoprotein E4 (APOE4) genotype exhibiting mild cognitive impairment (MCI) (See Patent Document 1).

To objectively assess Alzheimer's disease (AD), we chose to evaluate neural quality in patients with Alzheimer's disease using advanced magnetic resonance imaging technology using a modified diffusion tensor imaging (DTI) procedure.

For explanation, we note that MRIs of the brain were performed at the radiology department of Nishijima Hospital, and the results were sent to the doctors via an electronic charting system. Digital tractographic imaging was performed using Neuro3D and GRAPPA technology, with five starting points set at locations where nerve bundles passed through the entire hippocampus. DTI was obtained at fractional anisotropy (FA) values of 0.1 and 0.2. The size of the pathway (tract) was calculated from the number of pixels in the pathway (tract) display, the number of pixels was calculated using Image J software. DTI at a fractional anisotropy (FA) value of 0.1 reflects visualization of the neuron as a whole, DTI reflects at a fractional anisotropy (FA) value of 0.2 to the neural quality of the height visualisation. This is because, if neural quality is maintained, DTI shows that water molecules diffuse along the nerve fibers in only one direction
(see Patent Document 2).

### Documents known from the background art

### Patent literature

Patent Document 1: JP2019209018
Patent Document 2: WO2018/012596

### Description of the invention

### Summary of the invention

The purpose of the present invention is to develop a drug/treatment agent that maintains cognitive improvement and neural improvement/enhancement for a defined period of time even after the treatment is completed. In other words, it is the development of a causal/cause-oriented treatment. In addition, it is the development of an ameliorative drug/treatment whose purpose is to improve quality of life.

### Methods of solution

The number of Alzheimer's patients is increasing rapidly worldwide. The drugs and medicines that are currently available for the treatment of Alzheimer's disease show moderate efficacy and provide only temporary relief from clinical problems (symptomatic therapy). Drugs and medicines that slow down the progression of the disease do not have a significant effect, and their side effects cannot be ignored. No matter how many resources and efforts have been put into the development of drugs for Alzheimer's disease over the past 30 years, no drug has been developed that can slow or stop the progression of the disease, or that can effectively improve the symptoms (disease modifying therapy). Causal treatment means that the effect of the treatment persists for a specified period of time after the treatment has been stopped. Causal treatment is also called causal therapy, rootoriented treatment, etc.

Fecal incontinence causes confusion for patients and increases the burden on caregivers. At this stage, caregivers desire to use hospice care or other support services that focus on providing comfort and dignity in the final period of life. However, as the number of patients increases, the lack of facilities providing such adequate care becomes a serious problem. Incontinence, particularly loss of defecation control, is the most common reason for placing these patients in facilities. It is hoped that if a new treatment method could improve fecal incontinence in patients with progressive Alzheimer's disease, it would be possible to care for them in their home environment.

The inventors found that hydrogen gas made causal/cause-oriented treatment of Alzheimer's disease possible and that it could solve problems associated with fecal incontinence, etc., which led them to complete the invention.

The present invention is as follows:
(1) A causally acting drug containing hydrogen gas as the active substance for use in the treatment of Alzheimer's disease (disease-modifying drug).
(2) A causally acting drug (1) with a sustained therapeutic effect for at least 6 months after the end of treatment.
(3) A causally acting drug (1) with a sustained treatment effect one year after treatment compared to pre-treatment status.
(4) An enhancement drug containing hydrogen gas as an active ingredient that improves the quality of life of Alzheimer's patients.
(5) An enhancement drug containing hydrogen gas as an active substance improving nerve function and with a therapeutic effect even in patients with a severe form of Alzheimer's disease.
(6) An enhancement drug containing hydrogen gas as an active ingredient to improve fecal incontinence in patients with Alzheimer's disease.
(7) An enhancement drug containing hydrogen gas as an active ingredient to improve urinary incontinence in patients with Alzheimer's disease.

This description includes the publication of Japanese patent application No. 2022-122081, which confers priority to this application.

### Contribution of the invention

Hydrogen gas makes causal/cause-oriented treatment of Alzheimer's disease possible, and, in addition, it can improve fecal and urinary incontinence in Alzheimer's patients.

### Brief description of diagrams/circ.

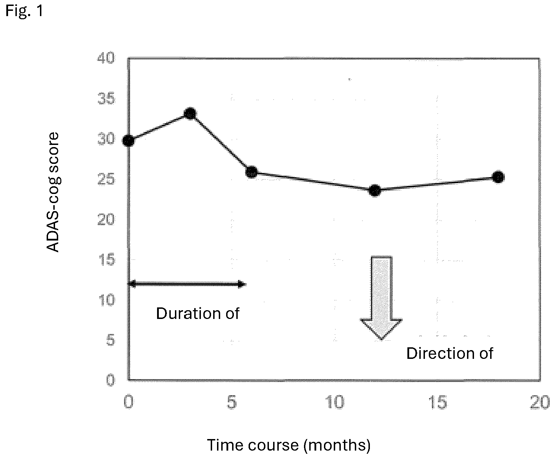

Fig. 1 shows the average change in clinical symptoms over time in eight patients with Alzheimer's disease as assessed by the ADAS-cog. Hydrogen gas inhalation was performed from 0 to 6 months, after which inhalation was discontinued. Monitoring of the course showed that the improvement persisted for 6 months or even 1 year after the cessation of inhalation.

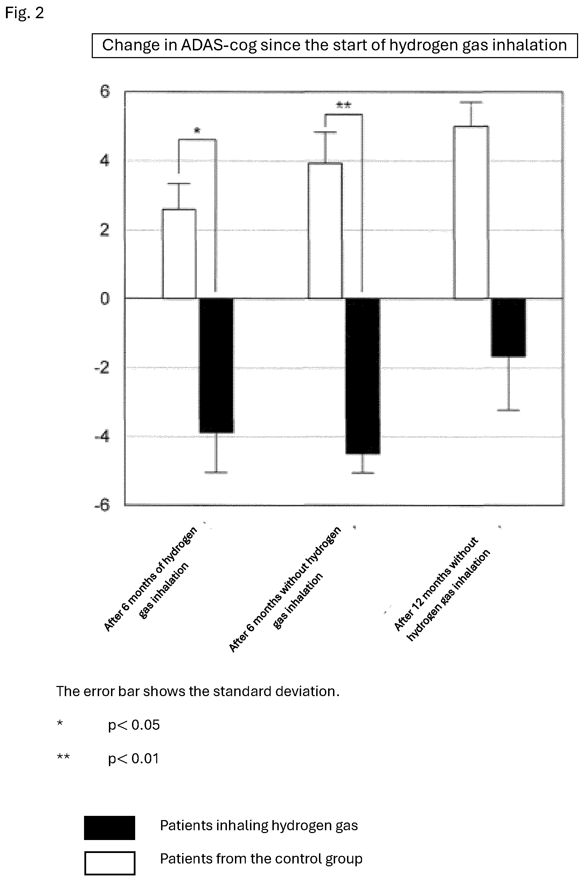

Fig. 2 shows a graph comparing the changes in ADAS-cog scores after initiation of hydrogen gas inhalation versus a control group of patients who did not inhale hydrogen gas. Follow-up showed that the improvement persisted for 6 months after cessation of hydrogen gas inhalation, and even one year after cessation of treatment, the improvement persisted compared to the pre-treatment status.

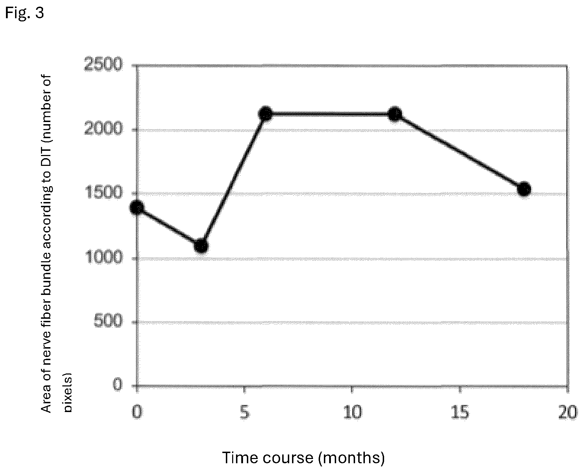

Fig. 3 shows, via DTI, the average change in neural quality over time in 8 patients with Alzheimer's disease. Hydrogen gas inhalation was performed from 0 to 6 months, after which inhalation was discontinued, and the course was monitored. At 6 months, the DTI area of the neural pathways increased and neural quality improved. After discontinuation of inhalation, the effect persisted for another 6 months, and even after 1 year after discontinuation, improvement persisted compared with the pre-treatment state.

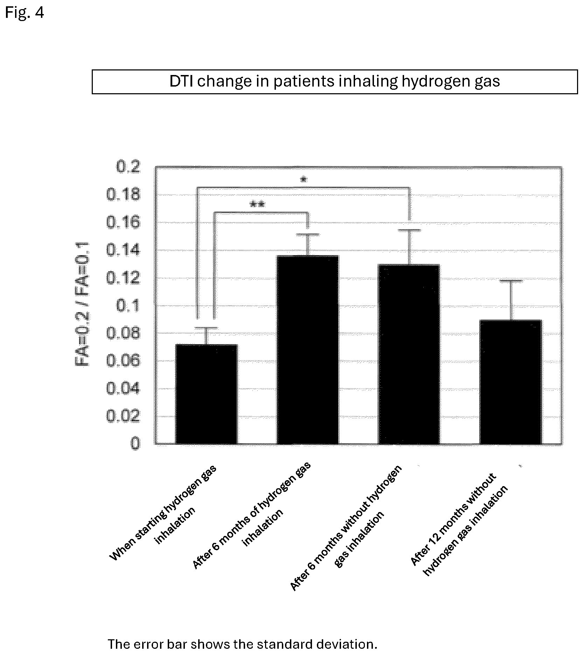

Fig. 4 shows a graph with the statistical analysis performed, where for statistical analysis reasons the variance of the individual measurements was improved to obtain more accurate values and the value of FA=0.2 was normalised to FA=0.1 (FA=0.2/FA=0.1). Hydrogen gas inhalation showed a significant effect after 6 months, with the significant effect persisting 6 months after the end of treatment, and the beneficial effect, compared with the pre-treatment condition, persisting after 1 year.

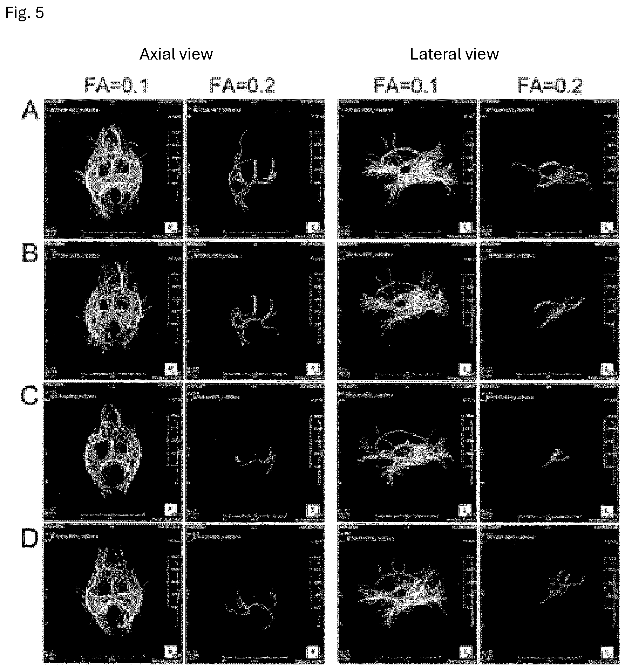

Fig. 5 shows the changes in DTI over time in one patient with Alzheimer's disease. In this patient, the effect did not appear after 6 months of hydrogen gas inhalation, so hydrogen gas inhalation was continued. After 2 years, the area of nerve fibers visualized in the DTI increased at FA=0.2. Axial view shows anterior view, lateral view shows lateral view.

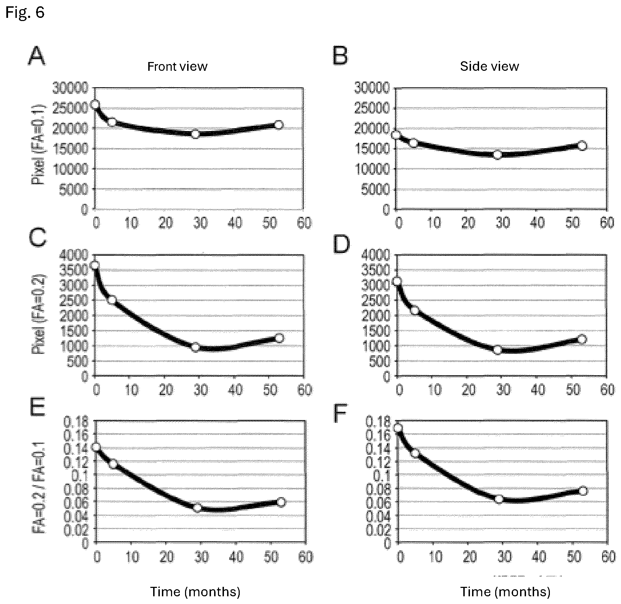

Fig. 6 quantifies the nerve fiber bundle area from Fig. 5 and shows the change over time.

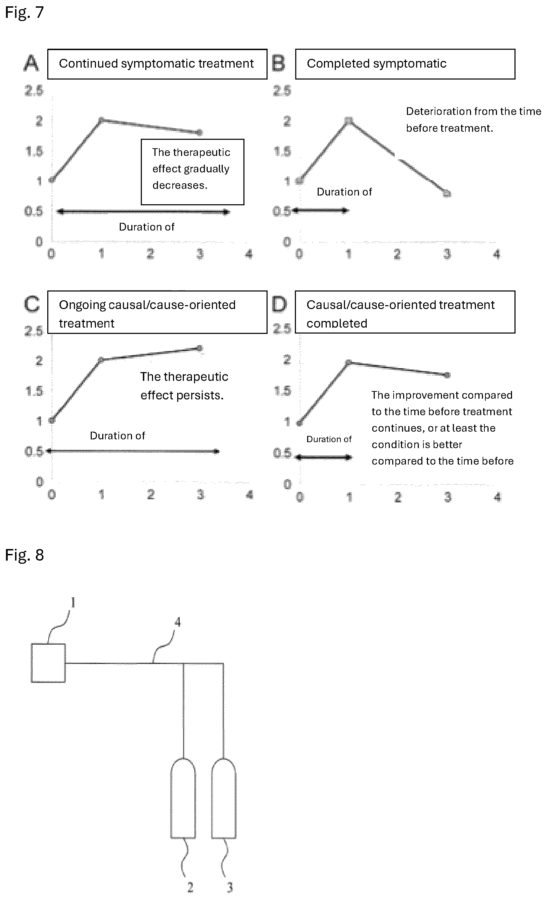

Figure 7 shows the differences between disease-modifying therapy and symptomatic therapy.
Fig. 8 shows a schematic diagram of a device designed to administer hydrogen gas to patients.

### Methods of implementing the invention

Below is a detailed description of this invention.

The present invention is a causally acting drug for use in the treatment of Alzheimer's disease. Causal treatment refers to a fundamental treatment, a sustained therapeutic effect after the treatment is completed. For example, that the improvement in Alzheimer's disease persists 3 months after the end of treatment, or 6 months, or 1 year, or the improvement persists after 2 years. Fig. 7 shows the differences between causal/cause-oriented and symptomatic treatment. The horizontal axis of the graph in Fig. 7 shows the duration of treatment, the vertical axis shows the improvement achieved by the patients (the higher the number, the greater the effect). As shown in Figure 7, with symptomatic treatment, even with continued therapy, the effect decreases, and there is also a worsening after treatment compared to the pre-treatment state. In contrast, causal treatment produces a sustained effect with continued treatment, which persists after treatment is stopped, or at least an improvement over the pre-treatment state.

Through this causal/cause-oriented drug according to the present invention, it is possible to improve fecal and urinary incontinence in patients with Alzheimer's disease. In other words, patients with Alzheimer's disease cannot control defecation and micturition, for example, they find it difficult to go to the toilet independently and therefore experience fecal and urinary incontinence, but with treatment using a cause-oriented drug containing hydrogen gas as the active agent, they can go to the toilet and defecate. In other words, the causally acting drug according to the present invention improves fecal incontinence and urinary incontinence in patients with Alzheimer's disease. A causally acting drug for use in the treatment of Alzheimer's disease according to the present invention may also improve the quality of life of Alzheimer's disease patients. A causally acting drug for use in the treatment of Alzheimer's disease according to the present invention also has an effect on patients with a severe form of Alzheimer's disease. Patients with severe form of Alzheimer's disease are, for example, patients with a high degree of progression (grade 7a to 7f) of Alzheimer's disease (FAST, Sclan SG et al. Int Psychogeriatrics, 1992; 4 Suppl 1: 55-69).¹
¹ Reference to the source doi: 10.1017/s1041610292001157 - note to source.

The concentration of hydrogen gas contained in a causally acting medicinal product containing hydrogen gas as the active substance for use in the treatment of Alzheimer's disease is 1 to 4 % (v/v) or 2,5 to 3,5 % (v/v) or approximately 3 %. The hydrogen gas content should not exceed approximately 4 % (v/v) for safety reasons, however, it is possible to have a higher hydrogen gas content under leak-tight conditions to avoid the generation of static electricity. Causally acting medicinal product containing hydrogen gas as the active ingredient for use in the treatment of Alzheimer's disease according to the present invention may comprise oxygen gas and/or other inert gases. The oxygen gas composition comprises a mixture of hydrogen gas and oxygen gas. The gaseous oxygen is consumed during respiration. The concentration of gaseous oxygen is 10 to 30 % (v/v), 15 to 25 % (v/v) and 21 % (v/v), respectively. Of the inert gases, nitrogen, helium, argon, etc., can be used; inexpensive nitrogen is suitable. The volume of this inert gas may be determined by the skilled person at his discretion within a range that is not too high, with regard to the concentration of gaseous oxygen for respiration, up to 80 % (v/v) or less is preferable.

For a causally active medicinal product containing hydrogen gas as the active substance for use in the treatment of Alzheimer's disease according to the present invention, a mixture of hydrogen gas with air may also be used. Such a gaseous mixture can easily be produced by adequately mixing air and hydrogen gas. Furthermore, gases used for anesthesia can be used as a possible application. In such a case, the causally acting drug comprising hydrogen gas as the active agent for use in the treatment of Alzheimer's disease according to the present invention will be a mixed gas comprising hydrogen gas and an anesthetic gas. The anaesthetic gas may be, for example, paradise gas (nitrous oxide).

A causally acting drug comprising hydrogen gas as an active agent for use in the treatment of Alzheimer's disease according to the present invention can be stored in pressurized containers, e.g. gas cylinders. Also, a device generating hydrogen gas by electrolysis of water can be used. The present invention also includes a container for a causally acting drug comprising hydrogen gas as an active agent for use in the treatment of Alzheimer's disease and a device/apparatus for enabling the generation of hydrogen gas.

A subject may use this causally active drug containing hydrogen gas as the active ingredient for use in the treatment of Alzheimer's disease according to the present invention, can be inhaled. The inhalation may be accomplished by means of an inhalation device utilizing a delivery line from a container containing a causally active drug comprising hydrogen gas as an active agent for use in the treatment of Alzheimer's disease according to the present invention. The method of inhalation is not limited, for example, an inhalation mask may be provided, it being desirable that such a mask covers the patient's mouth and nose simultaneously. A nasal tube may also be used to inhale hydrogen gas. A sealed chamber of adequate size to accommodate the patient may also be used for inhalation of hydrogen gas by the patient, and while in the chamber the patient may inhale into the chamber a causally acting drug comprising hydrogen gas as an active agent for use in the treatment of Alzheimer's disease according to the present invention. Such a chamber is one example of a patient being able to inhale a causally acting drug comprising hydrogen gas as an active substance for use in the treatment of Alzheimer's disease according to the present invention while lying down or sitting.

The present invention further comprises a device for administering a causally acting drug comprising hydrogen gas as an active agent for use in the treatment of Alzheimer's disease according to the present invention to patients with Alzheimer's disease, [namely] a container containing a causally acting drug comprising hydrogen gas as an active agent for use in the treatment of Alzheimer's disease, a gas inhalation device, and the aforementioned container equipped with delivery tubes from which the gas to be inhaled is delivered. Such a container may be, for example, a hydrogen gas cylinder. Any method may be used to obtain the hydrogen. Hydrogen may be generated by electrolysis of water, photocatalytic splitting of water, extracted from hydrogen generating materials, hydrogen storage/absorbing alloys, etc. Similarly, as mentioned above, an inhalation mask, a nasal tube can be used to inhale the gas, and a sealed chamber was mentioned. The method of hydrogen inhalation can be any method. The device is preferably provided with a container containing at least one gas from the gas group oxygen, inert gases, air, anaesthetic gas, in which case it is advantageous to supply a causally acting drug containing hydrogen gas as an active agent for use in the treatment of Alzheimer's disease together with at least one selected gas from the gas group oxygen, inert gases, air, alone or in a mixture. A nebulizer may also be used. For example, a suction bag can be attached to the inhalation mask, into which hydrogen gas can be supplied from a gas bottle containing hydrogen. A schematic diagram of a device according to the present invention is shown in Figure 8. The diagram shows an inhalation device (1), a container (2) containing a medicinal substance comprising hydrogen gas as an active ingredient, a container (3) containing at least one gas of the group oxygen, inert gases, air, anaesthetic gas and a supply tube (4) through which the gas is fed to the inhalation device and administered to the patient. In the case of nasal tube inhalation, it is not necessary to pre-dilute the hydrogen gas with other gases; the hydrogen gas can also be diluted with air during breathing.

The administration of a causally active drug comprising hydrogen gas as the active agent for use in the treatment of Alzheimer's disease is preferably a single administration of 0.1 h to 5 h, more preferably 0.3 h to 2 h, or administration for 1 h 1× to 5× , or 1× to 3× , or 2× , over a period of 1 to 30 days, or 5 to 20 days, or 6 to 10 days, respectively. The rate of inhalation when administering hydrogen gas depends on the lung capacity of the individual, for example, several litres per minute, or approximately 8 litres.

The effect of a causally acting drug containing hydrogen gas as an active agent for use in the treatment of Alzheimer's disease according to the present invention can be assessed by the Alzheimer's Disease Assessment Scale-cognitive subscale (ADAD-cog) cognitive assessment tool (Rosen WG, Mohs RC, Davis KL (1984) A new rating scale for Alzheimer's disease. Am J Psychiatry 141, 1356-1364). The ADAS-cog assesses and scores word list equipment, speaking ability, verbal comprehension, speech difficulties in spontaneous speech, completion of commands, object and finger naming, constructional tasks, ideomotor skills, orientation, word recognition, and ability to reproduce test instructions. The range of scores is from 0 to 70, the higher the score, the greater the cognitive impairment (normal→ several).

For example, if a subject diagnosed with Alzheimer's disease, in particular progressive Alzheimer's disease, or is administered, a causally active drug comprising hydrogen gas as an active agent for use in the treatment of Alzheimer's disease according to the present invention, the overall score on the ADAS-cog tool will be improved, e.g. the score [in the subscale] of the word list equipment will improve by one point or more, or by two points or more (the value of the score achieved will decrease).

It is also possible to assess the effect of a causally acting drug by neural quality. In this case, the neural quality can be assessed based on the area image obtained by diffusion tensor imaging (DTI) technology using advanced magnetic resonance imaging of the brain.

For example, the progression of nerve fibers in the hippocampal region of the temporal lobe of the subject's brain can be monitored using diffusion tensor imaging [DTI] and the area of nerve fibers monitored can be analyzed using the process procedures described below.
i) the process of generating a diffusion tensor imaging [DTI] image extracted from the course of nerve fibers in the hippocampal region of the temporal lobe of the subject's brain;
ii) the process of setting five control inputs at regular intervals so that the nerve fiber pathways extracted by process (i) pass through them;
iii) a process in which the fractional anisotropy (FA) is set to 0.2 and an image of the extracted nerve fibers in the hippocampus region is obtained;
iv) a process in which the area of the nerve fiber path is calculated from the number of pixels in the image obtained during the process iii);
v) the process of detecting the manifestations of dementia or predementia based on the measured values of the area of nerve fiber pathways.

In the method described above, the hippocampal region refers to the hippocampal region and the parahippocampal gyrus region; the extracted images of nerve fibers in the hippocampal region are from the lateral and axial views.

When the fractional anisotropy (FA) value is set to 0.1, the extracted image of nerve fibers in the hippocampus region is obtained, and in the process of calculating the area of nerve fiber pathways from the number of pixels in a given image, the area of nerve pathways at FA 0.2 is divided by the area of nerve pathways at FA 0.1 to calculate the FA ratio (normalization). Based on the FA ratio, neural quality can be assessed.

In (ii) above, 5 connected regions of interest (ROIs) can be created on the diffusion tensor representation.

The details of the method are described in International Application WO2018/012596.

### Examples

The present invention will be specifically described with reference to the following examples, but the present invention is not limited to these examples.

### [Example 1]

Hydrogen gas inhalation was performed in accordance with the World Medical Association Code of Ethics² (Declaration of Helsinki). The clinical trial protocol was approved by the ethics committee of Nishijima Hospital (2935-7, Ooka, Numazu Municipality, Shizuoka Prefecture) and was registered in the database URL: http://www.jmacct.or.jp under JMACCT ID: JMAIIA00308. The families also gave written informed consent.
² WMA (World Medical Association)

The inclusion criteria for patients with Alzheimer's disease were as follows:
(1) Patient diagnosed with Alzheimer's disease according to the National Institute on Aging (NIA)³ and Alzheimer's Association (AA)⁴ recommendation criteria.
(2) Patients with an ADAS-cog tool score greater than 10 and less than 50, or with a corresponding score recalculated by the MMSE tool using the 70- (MMSE× 2.33) formula.
(3) Patients retested every six months with the ADAS-cog/MMSE instrument as part of their routine treatment at a neurological dementia clinic.
(4) Patients without serious respiratory disease that could be expected to prevent effective inhalation of H₂ such as chronic obstructive pulmonary disease (COPD), pneumonia, airway inflammation, asthma, etc.
(6) Patients with magnetic resonance imaging (MRI) experience of the brain.
³ NIA: National Institute of Aging.
⁴ AA: Alzheimer's Association.

The total number of patients was 8, the proportion of women was 87.5%, the age was 79.4± 6.1 years (standard deviation), and the mean total ADAS-cog score was 229.7 ± 8.0 (standard deviation).

A control group of Alzheimer's patients who did not inhale hydrogen gas was assembled from past data of Nishijima Hospital patients. Initial scores on the ADAS-cog ranged from 10 to 50, and data from patients who had been assessed by the ADAS-cog instrument for at least one and a half years at Nishijima Hospital were used simultaneously. The mean initial score of the 19 patients, along with the standard deviation, was 27.8± 3.5 (standard deviation).

A mixture of hydrogen gas 3% (v/v) and oxygen gas 21% (v/v) was administered to 8 patients with Alzheimer's disease for 1 hour 2× per day. Inhalation was continued for a period of 6 months, after which hydrogen gas inhalation was discontinued and progress monitored.

Treatment efficacy was assessed by clinical assessment using ADAS-cog and neural quality (integrity) was assessed by MRI using DTI.

Figure 1 shows the average change in clinical symptoms over time in eight patients with Alzheimer's disease as assessed by the ADAS-cog (the lower the score, the greater the improvement). Continuous inhalation of hydrogen gas for 6 months produced an initial deterioration, but soon showed an improvement in cognitive function. Six months or one year after the cessation of hydrogen gas inhalation, there was a marked improvement in cognitive impairment compared to the before treatment, the effect of hydrogen gas inhalation was sustained, in other words, the effect of treatment was continuous and sustainable.

Fig. 2 shows a graph comparing the differences from the control group not inhaling hydrogen gas, for each patient inhaling hydrogen gas. The graph shows the score ADAS-cog at the start of inhalation and the scores approximately 6 and 12 months apart. Statistical significance was assessed by Student's t-test, * indicates outputs for p< 0.05, ** for p< 0.01, indicating significant statistical significance.

Fig. 3 shows the evolution of DTI area over time at an FA value of 0.2 in 9 patients with Alzheimer's disease. Through DTI, it was shown that after 6 months of continuous hydrogen gas inhalation, neural quality was improved. Moreover, the effect of hydrogen gas inhalation persisted even 6 months after the cessation of hydrogen gas inhalation. Although the effect of hydrogen gas inhalation one year after the end of inhalation was weaker, it was able to maintain a favorable result compared with the condition at the start of inhalation.

For a more accurate quantitative analysis, normalization was performed for each measurement. Data variability was corrected by dividing the F=0.2 value by the simultaneous F=0.1 value. The number of pixels in the corresponding neuronal bundle pathway at FA=0.2 was normalized (divided) by the number of pixels at FA=0.1, and the mean and standard error were obtained from the normalized values from 4 lateral and axial images from the right and left hemispheres of each patient. Statistical significance was assessed by Student's t-test, * indicates outputs for p < 0.05, ** for p < 0.01, indicating significant statistical significance of the results.

In other words, as shown in Figures 1 and 3, the effect of hydrogen gas inhalation persists in the clinical picture and in the objective DTI not only after cessation of inhalation, but also with a delay 6 months after cessation of inhalation, and the results show an improvement from baseline even 12 months after cessation of inhalation. Moreover, as shown in Figures 2 and 4, not only was there a statistically significant effect after hydrogen gas inhalation, but the effect also remained statistically significant even 6 months after cessation of inhalation, the improvement persisted, compared to baseline at the start of hydrogen gas inhalation, even after 12 months.

### [Example 2]

A patient with a severe form of Alzheimer's disease with incontinence of stool and urine was given an inhaled gas mixture containing 3% (v/v) hydrogen gas and 21% (v/v) oxygen gas 2× daily for one hour. Clinical symptoms and, via DTI, improvement of neural pathways over time were monitored. After two years of hydrogen gas inhalation, the patient was able to urinate and defecate on his own.

In more detail, the patient was a 79-year-old woman who was diagnosed with typical Alzheimer's disease in 2011 and was administered Alzheimer's drugs such as Donepezil, Galantamine, Lithium, etc. The condition was progressively worsening, the ADAS-cog score was 48/70, it was a severe form of Alzheimer's disease.

The patient was unable to recall her date of birth at that time, but was able to walk to the toilet to urinate or defecate on her own. Due to the rapid deterioration of her condition, other treatments were not possible, so hydrogen gas inhalation was started as an adjunctive/complementary therapy on April 19, 2019.

The patient was unable to flush or use toilet paper at that time. On the side of fecal incontinence, there was no improvement in the frequency or severity of difficulties in January 2020.

On 11/18/2020, the patient's husband noticed that the patient had reached the toilet on her own and had sufficiently defecated on her own. Since then, she has been able to go and defecate on her own in almost all cases.

Figure 5 shows the time course of DTI. Fig. 6 shows the change in DTI area over time. The neural quality was assessed using advanced magnetic resonance imaging technology using the diffusion tensor imaging procedure. Fig. 5 shows the frontal and side views, the number of pixels neural area and Fig. 6 shows the change over time. In both cases shown Fig. 5 and Fig. 6, an increase in neural quality in the severe form of Alzheimer's disease is evident.

Reflecting the whole nerve at FA=0.1, it is clear from the number of pixels that there was a gradual deterioration over the period before hydrogen gas inhalation (5 to 29 months) (Figure 6 A and B). The value of FA=0.2 reflects the height of neural quality, whereas a decrease in pixel count indicates a decline in neural function (Figure 6 C to F).

After two years of inhalation, there was a slight increase in the number of pixels at F=0.1 and F=0.2, the increase was more noticeable at FA=0.1 (29 to 53 months) compared to FA=0.2 (Figure 6). These results indicate an improvement in neural quality after two years or so of long-term hydrogen gas inhalation. The display in Figure 5D at FA=0.2 shows that new nerves have appeared, demonstrating that there is not only a quantitative but also a qualitative improvement in neural quality. Similar to Example 1, the change in neural quality was demonstrated when the display was changed at FA=0.2.

### [Conclusion]

As a result, it was shown that 6 months of inhalation of a mixture of gases containing hydrogen gas improved the difficulties of Alzheimer's patients, and the effect of the treatment persisted even 6 months to 1 year after the cessation of hydrogen gas inhalation. Continuous inhalation of hydrogen gas for 2 years has been shown to improve even severe forms of Alzheimer's disease. Moreover, these inventions are based solely on clinical trials in which continuous clinical trials have involved the long-term administration of inhaled hydrogen gas to Alzheimer's patients; these results cannot be predicted from animal experiments, have not been described in the past, and cannot be easily inferred by experts in the art

### Industrial applicability

A drug comprising hydrogen gas as the active agent according to the present invention can be used for the causal treatment of Alzheimer's disease.

### Reference Signs List

- 1: Gas suction device
- 2: container containing hydrogen gas
- 3: A container containing optionally at least one gas from the gas group oxygen gas, inert gases or anaesthetic gases
- 4: tube

All publications, patents and patent applications mentioned in this description are cited directly in the text.

## Claims

1. A causally acting drug containing hydrogen gas as an active ingredient for use in the treatment of Alzheimer's disease (disease-modifying drug).

2. A causally acting drug according to claim 1, whose ameliorative effect lasts for at least 6 months after treatment.

3. A causally acting drug according to claim 1, wherein the ameliorative effect lasts for one year after the start of treatment compared to pre-treatment status.

4. An enhancement drug containing hydrogen gas as an active ingredient to improve the quality of life of patients with Alzheimer's disease.

5. An enhancement drug containing hydrogen gas as an active substance improving nerve function and with a therapeutic effect even in patients with a severe form of Alzheimer's disease.

6. An enhancement drug containing hydrogen gas as an active ingredient to improve fecal incontinence in patients with Alzheimer's disease.

7. An enhancement drug containing hydrogen gas as an active ingredient to improve urinary incontinence in patients with Alzheimer's disease.
